# EUROPEAN PATENT APPLICATION

(11) **EP 4 781 942 A1**
(43) Date of publication of application: **29.07.2026**
(21) Application number: 25153745.2
(22) Date of filing: 24.01.2025
(51) Int. Cl.: A61B 90/00, A61B 90/20

(54) **DEVICE FOR DISPLAYING SURGICAL IMAGES**

(71) Applicant: Leica Instruments (Singapore) Pte Ltd., Singapore 618299 (SG)
(72) Inventor: KOK, Alvin, 618299 Singapore (SG); ZOU, Qingsong, 618299 Singapore (SG); SIM, Yong Chin Eddy, 618299 Singapore (SG); PAN, Jiahao, 618299 Singapore (SG); LI, Michelle, 618299 Singapore (SG); LIM, Anthony, 618299 Singapore (SG)
(74) Representative: Paustian & Partner Patentanwälte mbB

(57) **Abstract**

A first aspect of the present disclosure is related to a device for displaying surgical images,
configured to:
- determine, via a content function, a first camera image and a second camera image based on a camera image of a surgical device ;
- determine, via a control function, one or more graphical user interfaces, GUIs, to control the surgical device and/or the content function;
- assign to a first display , in particular a touch screen display, the first camera image and a GUI concurrently;
- assign to a second display the second camera image and/or a GUI.

## Description

### Technical Field

This disclosure is related to devices and systems for displaying surgical images and methods therefor.

### Background

In surgical settings, the microscope's control interface often resides on a small touchscreen monitor in the rear of the operating room or near other systems, where space is restricted. This leads to several issues: accessibility is hampered by cramped placement, making it tough for operators to adjust settings. Training is hindered because multiple users cannot simultaneously view the small display. Additionally, reviewing media is constrained by limited screen space and how many people can access it. Improvements are desirable.

### Summary

An object of the present disclosure is to improve working with a microscope in a surgical environment.

This object is solved by the disclosed embodiments, which are defined in particular by the subject matter of the independent claims. The dependent claims provide information for further embodiments. Various aspects and embodiments of these aspects are also disclosed in the summary and description below, which provide additional features and advantages.

A first aspect of the present disclosure is related to a device for displaying surgical images,
*configured to:*
- *determine, via a content function, a first camera image and* a *second camera image based on a camera image of a surgical device ;*
- *determine, via a control function, one or more graphical user interfaces, GUIs, to control the surgical device and*/*or the content function;*
- *assign to a first display, in particular a touch screen display, the first camera image and* a *GUI concurrently;*
- *assign to a second display the second camera image and*/*or a GUI.*

A device for displaying surgical images can be a hardware device or a software device. Such a device can be a computer-based controller. Such a device can be a surgical microscope or a rack for a surgical microscope. Additionally or alternatively, such a device may comprise one or more displays and/or interfaces for one or more displays. Additionally or alternatively, such a device may comprise its own housing or may be integrated intro a surgical workstation and/or rack. Such a device may comprise, the first display, e.g., as a touch screen, and/or the second display.

An imaging device, by which surgical images may be generated, can be integrated into a microscope, a laparoscope, an endoscope, an arthroscope, an exoscope and/or other specialized surgical devices. Alternatively, imaging components may be attached to overhead operating lights or robotic surgical arms, providing surgeons with enhanced optical clarity and precision during minimally invasive or open procedures. An imaging device can comprise a plurality of imaging devices. In particular each imaging device may observe the surgery and/or a sample with different functionalities, e.g. a camera can provide a visual image and/or another camera can provide a fluorescence image, other cameras can provide a stereo image, an ultrasound image, an intraoperative X-ray image, and/or CT-image. An imaging device, e.g. a microscope, can be a standing tower or ceiling mounted.

A content function may be configured to generate a first camera image and a second camera image based on a camera image by the imaging device. The content function may be implemented in hardware and/or in software.

A control function may generate a graphical user interface (GUI) for one or more users. A control function may be utilized to configure and/or control a content function, i.e. functions of a content function. The one or more users can control the device, the first image, the second image, the provision of these images, and/or the imaging device over a GUI. A GUI may be configured to receive and/or process text commands and/or commands that are provided haptically (e.g. by touch), visually, and/or by gestures.

A first display can be a 2D or 3D display. A first display may be a touch screen display, i.e. an interactive electronic device that responds to finger or stylus input. A touch screen display may integrate visual output with tactile input, enabling a more direct, intuitive user experience. A touch screen display may use capacitive and/or resistive technology for measuring haptic inputs. A second display can be a 2D or 3D display. Additionally or alternatively, it can be a touch screen display as well. Both displays can be configured for an image stream, i.e. video display.

Based on the content function and the control function, image content and control functionality can be assigned according to a need of the users, i.e. surgeons and other personal, flexibly across different devices, such as the different displays.

An embodiment of the first aspect is related to a device for displaying surgical images,
*configured to:*
- *assign, by the content function, from one display to the other display the respective camera image and*/*or the respective GUI.*

Operating rooms may house multiple displays, each used toward showing distinct data, such as microscopic/endoscopic views, vital signs, or specialized imaging like MRI or CT scans. By swapping surgical images between these displays, visualization of critical information can be adapted to the viewers/users. One benefit is ensuring a clearer and/or larger view for a surgeon. Instead of crowding around a single monitor, the operating team can position an important feed at the center of attention, thereby improving visibility and focus. Additionally or alternatively, surgeons may observe a range of imaging modalities, e.g. ultrasound, intraoperative X-ray, or CT. This can be done in particular at different stages of a procedure. By swapping the relevant imagery to the most convenient monitor, they can transition fluidly between tasks while maintaining a cohesive workflow. Hence, different monitors may display different content information observed of the imaging device and/or from other sources (i.e. other imaging devices, stored images, etc.).

This approach is not constraint to monitors within an operator room. Camera feeds may be routed to a display visible to residents, students, or remote experts, outside an operating room who can then follow and guide the procedure without physically impeding the surgeon's workspace.

Swapping images may also reflect realities of equipment constraints. Displays can malfunction or fail to deliver the required resolution for certain procedures, prompting a rapid switch to another monitor to maintain continuity. Additionally or alternatively, having the ability to shift an imaging feed and/or GUI functionalities to a recording or archiving station may support legal, research, or quality-improvement documentation. By ensuring the right image appears on the right screen at the right time, operating rooms can maximize both surgical precision and overall procedural effectiveness. An image and/or GUI can also be swapped to another than the first or the second display, e.g., to a headset display and/or to a handheld display.

Assigning a GUI from one display to another display means that the functionality of the GUI is assigned from one display to another and not necessarily the visual appearance of the GUI. The latter can change, in particular depending on the display format leading to another resolution, another form of GUI-elements, another arrangement of GUI-elements and/or a selection of GUI-elements.

An embodiment of the first aspect is related to a device for displaying surgical images,
*configured to:*
- *determine, by the content function, the first camera image in a first resolution; and*
- *determine, by the content function, the second camera image in a second resolution.*

For example, a content function can configure the first camera image as a smaller image, with a lower resolution, to be displayed on a small touch screen display, e.g., used interactively by a surgeon. Additionally, the content function can configure the second image as an image to be displayed on a larger screen, e.g. for personal observing the surgical procedure, in a larger resolution than the first camera image. This can also be done the other way around, such that the content function provides an image to a, in particular larger, touch screen for assisting personal or an observing audience, and a second image is provided to a 2D screen for a surgeon.

An embodiment of the first aspect is related to a device for displaying surgical images,
*configured to:*
- *provide, by the content function, the first camera image to the first display within an image stream with a first refresh rate;*
- *provide, by the content function, the second camera image to the second display within an image stream with a second refresh rate, wherein the first refresh rate is different from the second refresh rate.*

Refresh rates may in particular be adapted to ensure real-time capability. In particular a refresh rate of a monitor for assistance and/or an audience may be lower than for a surgeon's display. Adapting refresh rates for two monitors (one dedicated to the surgeon and another for an audience) can enhance both real-time capabilities for the surgeon and/or overall viewing quality for observers. For the surgeon's display, a primary goal may be minimal latency and/or maximum responsiveness. Therefore, it is best to set a higher refresh rate (e.g., 60 Hz or 120 Hz) so that the live video feed updates as smoothly and quickly as possible. This may help a surgeon to interpret visual cues in real time and reduces input-to-display lag, which is critical when performing precise maneuvers.

Additionally or alternatively, the audience monitor can operate at a lower or different refresh rate. This may reflect the fact that teaching/informing observers may not require the same ultra-low-latency feedback. A refresh rate between 30 Hz or 60 Hz may be sufficient for general viewing or educational demonstration purposes. Allowing the audience feed to refresh at a rate that matches typical presentation standards also helps ensure compatibility with common video broadcasting equipment (e.g., projectors, recording devices, and streaming encoders), and can reduce the load on the system's video distribution hardware.

In another embodiment, the refresh rate of the camera image stream for the surgeon may be lower than the refresh rate for the audience. This may reflect the fact that the surgeon is provided with a modality that is captured by the camera at a low refresh rate, e.g. an ultrasound image. Additionally or alternatively, the surgeon may primarily observe the surgery directly and only resorts to the display for certain additional information. Then also a lower refresh rate for the surgeon's image may suffice.

If both feeds originate from the same source, the device according to the first aspect may output signals at different resolutions and/or refresh rates simultaneously. This means, the haptic display (e.g. the surgeon's monitor) may receive a high-refresh, low-latency signal, while the second display (e.g. for the audience) receives a second signal that may incorporate buffering and/or compression.

An embodiment of the first aspect is related to a device for displaying surgical images,
*configured to one or more of the following:*
- *provide, by the content function, one or more camera images to a storage for later use;*
- *obtain, by the content function, one or more camera images from a storage for current use;*
- *provide, by the control function, one or more GUI-user-interactions to a storage for later use;*
- *obtain, by the control function, one or more GUI-user-interactions from a storage for current use.*

An image can be stored as a raw image, i.e. before being processed to the first and/or for the second camera image. This allows adaptation of the raw image later when the raw image is actually used for display. Additionally or alternatively, an image can be stored concurrently to a provision of a first and/or a second camera image. For example, a first and a second camera image can be provided and concurrently the raw image, on which the first and the second camera images are based on may be stored. Alternatively, a first and/or a second camera image may be stored and provided to a display on the same time. In this case, a camera image, which was actually displayed, can be provided again.

A stored image can be fetched and provided to one or more displays. In particular, a stored image can be provided, e.g. to the surgeon, in order to give the user an information about a previous state of the observed sample (e.g. the patient, an organ, etc.). Therefore, a display can switch an actual camera image to the stored camera image. Additionally or alternatively, the actual camera image and the stored camera image may be displayed together on a display (on the display of the surgeon and/or the display of the audience). This can be done, e.g. displaying both images next to each other and/or by overlaying both images. In particular, the control function can provide GUI functionalities to fetch and display a stored image as needed.

Additionally or alternatively a GUI-user-interaction may be stored for later use and/or obtained from a storage for a current use. This may in particular facilitate training of surgeon's or surgical personal. A GUI-user interaction can be an image sequence, in which the impact of a user interaction on a GUI is shown, e.g. the GUI is shown with the different input elements changing because of the user interaction.

An embodiment of the first aspect is related to a device for displaying surgical images,
*configured to:*
- *assign to the second display, by the content function, the second camera image and, by the control function, a GUI with a functionality of the GUI that is assigned to the first display* .

In this embodiment the same GUI-functionalities are displayed on both displays. This may relate to the functionalities itself and not to the graphical presentation of the functionalities. Hence, in particular if the first display is a touch screen display, a GUI may be displayed graphically different than a GUI on the second display, but with the same functions. Alternatively, the GUI displayed on a touch screen display and on the second display are completely the same, i.e. they have the same functionalities and the same graphical structure, and they are only adapted for the remaining difference(s) of both displays, e.g. a different refresh rate and/or a different resolution.

An embodiment of the first aspect is related to a device for displaying surgical images,
*configured to:*
- *assign, by the control function, to the second display a GUI, which is different from the GUI assigned to the first display* .

A multi-screen surgical system can be beneficial when each display is tailored to a specific purpose or workflow. For instance, the primary screen might present critical real-time imaging and/or surgical controls intended for a lead surgeon, thereby keeping the immediate procedure in focus. Additionally, the second screen could be dedicated to tasks like monitoring patient vitals, reviewing medication logs, or displaying procedural checklists for the anesthesiologist, nurse, or surgical assistant. The different GUIs displayed at the two screens may reflect the different purposes and functionalities.

Additionally or alternatively, this approach supports task separation and reduces cognitive load. Users may be allowed to enter data and/or adjust system settings on the second display without disrupting a primary surgical view on a first display for the surgeon. By isolating less urgent information, the team can maintain continuous awareness of essential data while still accessing necessary details. In terms of workflow, having distinct GUIs also facilitates parallel processes. For example, real-time anatomy tracking could run on one display, while patient metrics and imaging device's operating functionalities are displayed on the other display. Therefore, the GUIs for the different screens may be different.

An embodiment of the first aspect is related to a device for displaying surgical images,
*configured to:*
- *assign, by the control function, an image of the GUI and*/*or a GUI-user-interaction of the first display to the second display.*

This can be useful in particular in training scenarios, where an audience, which observes a surgery on a second display, can see the same GUI-inputs a surgeon or someone near the surgeon performs on a touch screen display. The audience may see on the second screen a different image, e.g., a larger image and/or an image enhanced with additional information, as the surgeon on the first display. The audience sees on the second display the GUI and the input/output information by the surgeon provided to the first display.

An embodiment of the first aspect is related to a device for displaying surgical images,
*configured to:*
- *to provide the first camera image directly from the camera to the first display and*/*or to the second display.*

Providing the first camera image directly from the camera to one or both of the displays may reduce latencies and may assure realtime capability. Because the images are not routed over other nodes and therefore communication time can be reduced.

An embodiment of the first aspect is related to a device for displaying surgical images,
*configured to:*
- *receive a selection information* , *by the control function, of an object and*/*or an area indicated on one of the first display or the second display;*
- *provide the selected object and*/*or area for display, by the content function, to the respective other of the first display and*/*or the second display.*

Limiting a second display to only show a chosen section (a selected object and/or area) of a surgical image can improve focus. The format/size of the first or the second camera image may be provided depending on the selection information as selected in the other of the first or the second image (on the respective first or second display). Surgeons may need to concentrate on a particular surgical area, so cropping out unrelated content may reduce distraction and clarify details. On the same time, the cropped image can also be shown to an audience on a second screen. In educational environments, partial views help instructors guide trainees by demonstrating key maneuvers on a screen for an audience. Hence, the selected information can also be provided on a screen not intended for the surgeon, but for an audience.

The selected object and/or area can be selected, e.g. via a GUI provided by the control function. Then the information about the selection can be communicated to the content function, such that the image can be provided in a desired configuration (format, resolution, refresh rate, etc.) such that the information is adapted to the intended display. The desired configuration can also be provided by the control function and in particular gathered from a user input. Transmitting only a selected information may conserve bandwidth, in particular in telemedicine.

The selected information may be indicated in the other screen, such that the user who receives the larger image knows that other users view selected information.

An embodiment of the first aspect is related to a device for displaying surgical images,
*configured to:*
- *receive a selection information of an object and*/*or an area indicated by a user on one of the first display or the second display;*
- *indicate the selected object and*/*or area on the respective other of the first display and*/*or the second display* .

Similar to the preceding embodiment, the second image can be displayed on the second display in a format independent on the selection information, but with the selection object /area indicated in the second image. For example, a surgeon may focus on a specific selection on her/his display and an audience on a second display may see the full scale image, but with the selected view of the surgeon indicated. This embodiment may also facilitate multitasking: while one screen provides an overview of the entire operation, the other highlights instruments and/or anatomical regions that require precision.

An embodiment of the first aspect is related to a device for displaying surgical images,
*configured to:*
- *display a structure on the second display indicated on the first display;*
- *display an area of interest in a second image;*
- *display a past part of a surgery for replay.*

Displaying selected and/or interesting parts of the surgery may enhance communication between surgeons and other personal during a surgery.

A second aspect of the present disclosure is related to a system for displaying surgical images,
*comprising:*
   - *a device according to one of the preceding aspects,* - *a first display, in particular a touch screen display ;*
   - *a second display ;*
   - *an input means;*
*configured to:*
   - *assign results of a content function and*/*or of a control function of the device according to one of the preceding aspects based on an information provided by the input means;*
*wherein the input means comprise one or more of:*
   - *a haptic input means, in particular a footswitch;*
   - *a remote control;*
   - *a microphone;*
   - a *camera.*

With a system according to the second aspect of this disclosure an operating room can be equipped with flexible display functionalities for surgeons, surgical personal and/or third parties that perform and watch a surgical procedure.

A third aspect of the present disclosure is related to a method for displaying surgical images,
*comprising the steps:*
- *determining a first camera image and* a *second camera image based on a camera image of a surgical device ;*
- *determining one or more graphical user interfaces, GUIs, to control the surgical device and*/*or the determination of the first and*/*or second camera image;*
- *assigning to a first display, in particular a touch screen display, the first camera image and* a *GUI concurrently;*
- *assigning to a second display the second camera image and*/*or a GUI.*

A method according to the third aspect of this disclosure can comprise one or more steps to execute one or more functions of a device according to the first aspect and/or a system according to the second aspect of this disclosure. Steps of a method according to the third aspect of this disclosure can be configured such that they comprise features of a device according to the first aspect of this disclosure. In particular, a method according to the third aspect of this disclosure can comprise steps for operating a device according to the first aspect and/or a system according to the second aspect of this disclosure.

A fourth aspect of the present disclosure is related to a computer program with program code,
*for operating a device,* a *system and*/*or for executing a method according to one of the preceding aspects, when the computer program is run on a processor.*

The computer program can comprise program code for providing one or more functionalities of a device and/or a system according to the first and second aspect of this disclosure. Additionally or alternatively, the computer program can provide program code for executing one or more steps of a method according to the third aspect of the present disclosure.

A further aspect of the present disclosure is related to a computing device comprising a processor configured to carry out the method according to any one of preceding aspects/embodiments.

A further process of the present disclosure is related to a computer program product comprising instructions which, when the program is executed by a computer system, cause the computer system to carry out the method according to any one of the preceding aspects/embodiments.

A further aspect of the present disclosure is related to a computer-readable medium comprising instructions which, when executed by a computer system, cause the computer system to carry out the method according to any one of the preceding aspects/embodiments.

### Brief description of the figures

Further advantages and features result from the following embodiments, some of which refer to the figures. The figures do not always show the embodiments to scale. The dimensions of the various features may be enlarged or reduced, in particular for clarity of description. For this purpose the figures are at least partially schematized.
Fig. 1 illustrates a microscope system according to an embodiment of this disclosure.
Fig. 2 illustrates a first camera image for a first display with Content and GUI according to an embodiment of this disclosure.
Fig. 3 illustrates a GUI for a second display according to an embodiment of this disclosure.
Fig. 4 illustrates display of a first camera image on a first display and a second camera image on a second display in an operating room during a surgery, according to an embodiment of this disclosure.
Fig. 5 illustrates display of a first camera image on a first display and a second camera image on a second display in an operating room during a surgery, according to an embodiment of this disclosure.
Fig. 6 illustrates display of a first camera image on a first display and a second camera image on a second display in an operating room during a surgery, according to an embodiment of this disclosure.
Fig. 7 illustrates a microscope system according to or for embodiments of this disclosure.

Although some aspects have been described in the context of an apparatus (or a system) in the present disclosure, the description of these aspects also represents a description of the corresponding method, where a block or device corresponds to a method step or a feature of a method step.

Analogously, aspects described in the context of a method step also represent a description of a corresponding block, item, or feature of a corresponding apparatus or of a system that may in particular be distributed over different locations and is configured to exchange information between the different locations with respective communication means.

In general, a disclosure of a described method also applies to a corresponding device (or apparatus) for carrying out the method or a corresponding system comprising one or more devices and vice versa. For example, if a specific method step is described, a corresponding device may include a feature to perform the described method step, even if that feature is not explicitly described or represented in the figure. On the other hand, if, for example, a specific device is described on the basis of functional units, a corresponding method may include one or more steps to perform the described functionality, even if such steps are not explicitly described or represented in the figures. Similarly, a system can be provided with corresponding device features or with features to perform a particular method step. The features of the various exemplary aspects and embodiments described above or below may be combined unless expressly stated otherwise.

As used herein the term "and/or" includes any and all combinations of one or more of the associated listed items and may be abbreviated as "/". Expressions as, "for example", "e.g.", or "in particular" denote facultative or optional features that can be combined with all other (mandatory, facultative, or optional) features of the aspects or embodiments of this disclosure, until explicitly stated otherwise.

In the following description reference is made to the accompanying figures which form part of the disclosure and which illustrate specific aspects in which the present disclosure can be understood. Identical reference signs refer to identical or at least functionally or structurally similar features.

### Detailed description

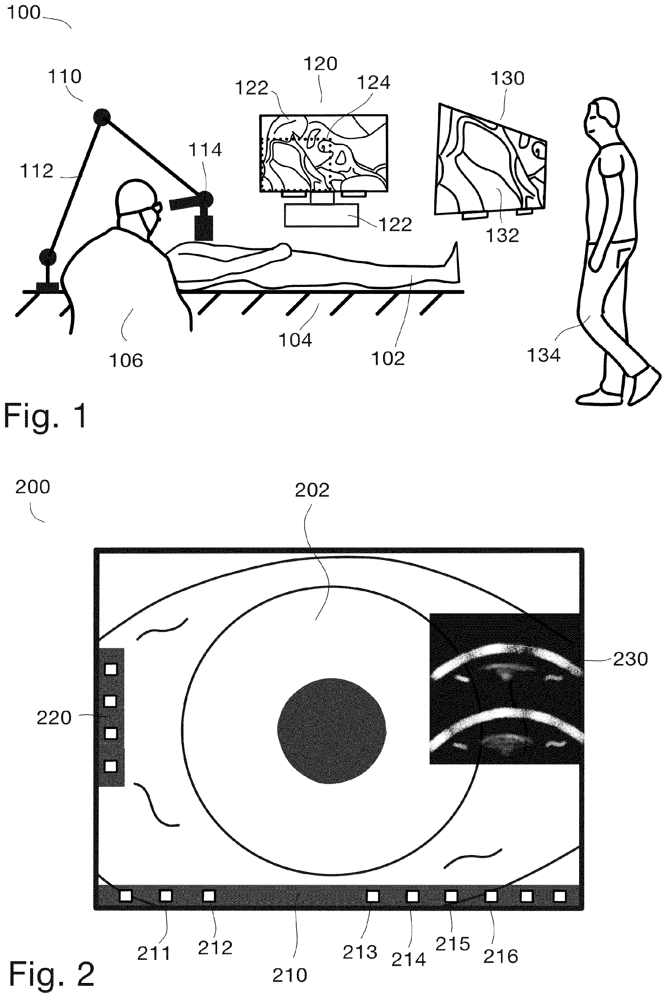

Fig. 1 illustrates a microscope system for an operating room 100 according to an embodiment of this disclosure. A patient 102 lies on an operating table 102 and a surgeon 106 scrutinizes an area (e.g. an eye) of the patient 102 over an articulated microscope 110. The articulated microscope 110 is mounted on the operating table 104 and movable by arm 112. At the tip of the arm 112 an optical system 114 of the articulated microscope is arranged such that it can be moved flexibly to observe the patient 102.

The microscope system 100 is equipped with two displays for images of the microscope 114. A first screen 120, which is a touch screen, is arranged such that the surgeon and its assistant can watch an first image 122 observed by the microscope 114. Therefore, a content function of the system configures the image information observed by the microscope 114 such that the information can be displayed in real-time at the touch screen 120. Therefore resolution is adapted and the image information is send directly to the touch screen 120 to minimized processing delays.

A control function provides a graphical user interface to the touch screen. By the GUI the surgeon or its assistant can select certain information in the first camera image 122 on the touch screen 120. The selected information 124 is communicated from the control function to the content function, such that the content function can generate an image based on the selection information 124. This image is then generated, sent, and displayed as second camera image 132 to the second display 130. The second display provided for an audience 134, e.g. further surgical personal or students. Based on the information from the surgeon, the audience can focus on specific parts of a surgery.

Additionally or alternatively, the content function may - in a first step - send the same image information to the second display 130 as to the first display 120. Display 130 may also comprise a GUI such that a user 134 may select a certain part 132 of the second image to be displayed. Information about the selection by the audience is then displayed on the first display 120 as selection information 124. In this case, the surgeon can follow on what part the audience focusses. The audience may in particular be a lead surgeon who does not perform the surgery by herself/himself, but who indicates to the operating surgeon certain important information by the selection 132/124.

Fig. 2 illustrates a first camera image 200 for a first display with image information and GUI information, according to an embodiment of this disclosure. The first camera image is based on a microscope image 202 (showing an eye of a patient), which is adapted by a content function to be displayed at a first screen. Additionally further information, e.g. ultrasound information, is provided as subimage 230. Additionally, a GUI is provided to be overlaid over the first camera image 202. The GUI comprises two graphical main elements. One part of the GUI is provided as a sidebar 220. And another part of the GUI is provided as a bottom bar 210. The bottom bar has a plurality of further GUI-elements to operate the microscope and/or select/configure image information to be displayed on the first and/or the second image.

A function for configuring a footswitch overlay is provided by GUI-element 211. Different visual enhancement (e.g. low-pass filter, high-pass filter, AI-based object detection) can be selected by GUI-element 212. GUI-element 213 is a toggle switch by which the user can select 2D or 3D display. Optical coherence tomography (OCT) of the microscope can be configured by GUI-element 214. Via GUI-element 215, the user can switch the GUI from touch screen display to a stand monitor. Therefore, the control function may adapt the GUI such that the GUI-elements are distributed for the larger stand screen. Furthermore, the GUI is changed such that the GUI can be operated by a mouse or another pointing device if the stand screen has no touch functionalities. Via GUI-element 216, the first camera image can be provided to further screens that are connected over an HDMI-port.

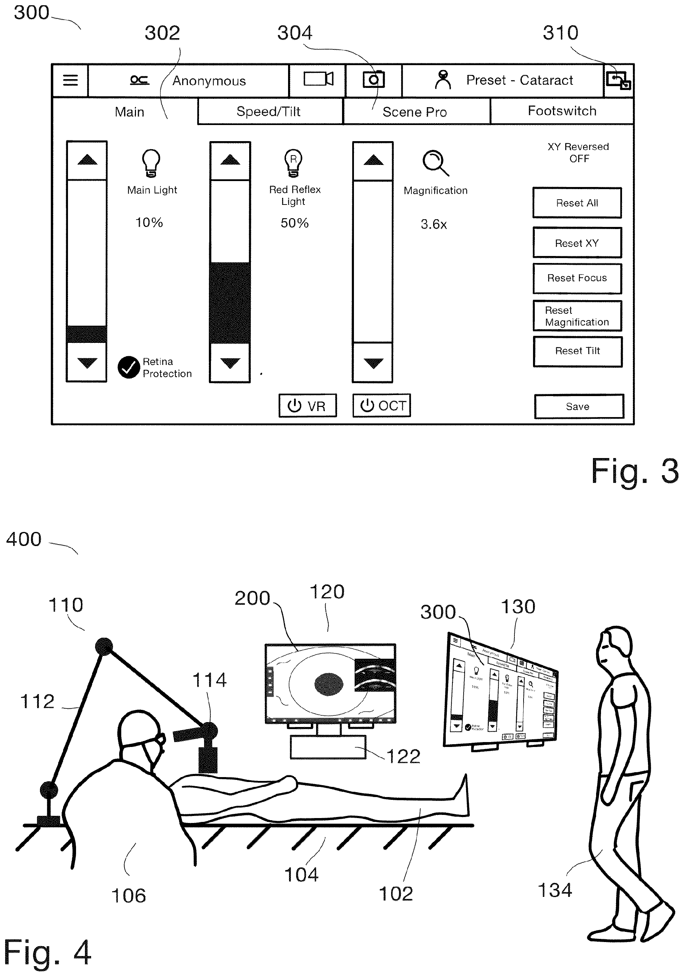

Fig. 3 illustrates a GUI 300 for a second display according to an embodiment of this disclosure. On the "Main" menu 302, the GUI 300 comprises GUI-elements to configure the surgical microscope. On the "Scene Pro" menu 304, enhancements for the first and/or the second (and further) camera images displayed on different screens can be configured. Furthermore, by GUI-element 310, the GUI can be swapped to the first display. The first display may have a different hardware configuration, e.g. a different size, resolution, refresh rate, and/or input capabilities. Therefore, the control function that controls the swap function, re-configures the GUI to adapt to the different hardware. Additionally, the control function may inform the content function to reconfigure the camera image to adapt to the different hardware. Adaptations may relate to the parameters named above.

Fig. 4 illustrates a display of a first camera image on a first display and a GUI on a second display in an operating room 400 during a surgery, according to an embodiment of this disclosure. The first camera image 200 originates from the microscope 114 and is similar to the image 200 of Fig. 2. It is displayed on a touch-sensitive display screen 120 which is configured to display the image to the surgeon and to assistant surgical personal. Additionally, on a second display 130 a GUI 300 similar to the GUI 300 described in relation to Fig. 3 is displayed. The GUI 300 comprises graphical elements to configure and control the microscope and the information displayed on the first display 120 and on the second display 130. The GUI 300 comprises a swap-screen element, by which the GUI is swapped to the first display 120. The second display can be a touch display on a microscope operating console. Alternatively, it can be a 2D-display configured for an audience that does not participate directly at the surgery.

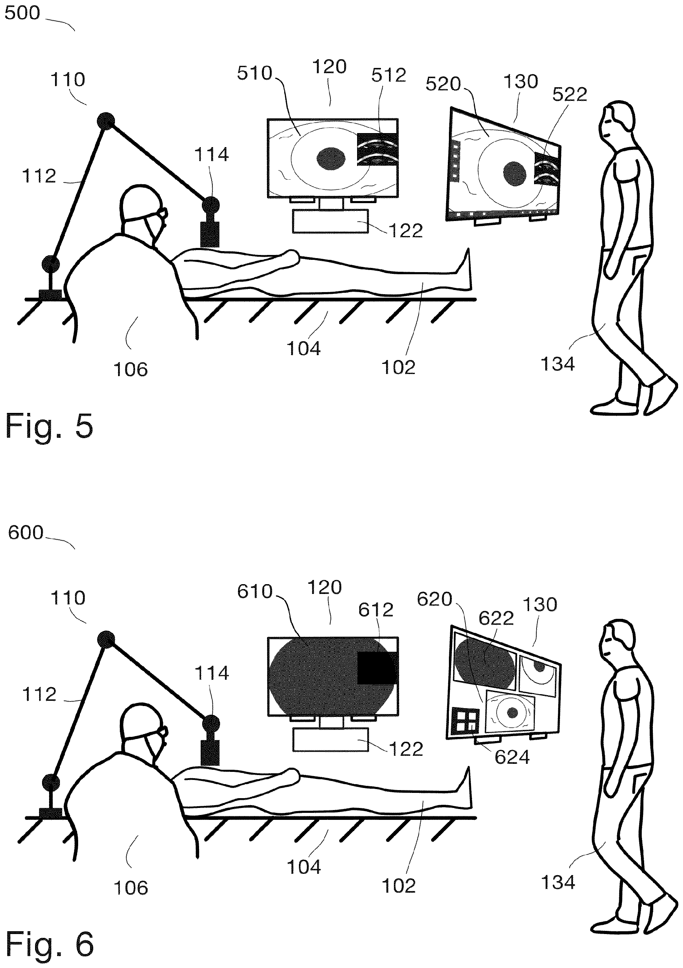

Fig. 5 illustrates a display of a first camera image on a first display and a second camera image on a second display in an operating room 500 during a surgery, according to an embodiment of this disclosure. In this embodiment, the first display 130 may be a display for an audience or a display for an operator 134 of the microscope 114, who supports a surgeon 106. The first display shows 130 a first image 520 produced by a content function from the microscope image and for the first display. Additionally, the image 520 comprises a GUI for operating the microscope. The GUI is generated for the first display by a control function. The content function further generated a second image 510 for a second display 120. The second display is intended to be used by the surgeon as the visual display of the microscope 114 (which has no display on its own). The content function further configured both images, the first image and the second image, to comprise an additional view 512, 522 information, which comprises additional information about the patient.

Fig. 6 illustrates a display of a first camera image on a first display and a second camera image on a second display in an operating room 600 during a surgery, according to an embodiment of this disclosure. In this embodiment, the first display 130 is fed by the content function with an image 622 of the microscope 114. The image is shown within a graphical user interface amongst other images, which show the operated organ in earlier states (previously captured, stored in a memory, and obtained therefrom by the content function) and/or which show other information. The displayed information can then be reviewed by a surgical team. Additionally, the second display 120 is fed by the content function also with the image 610 of the microscope 114, which is configured for display on the full size of the second display 120. The content function further feeds the first display with additional information to be shown as an overlay subimage 612 over the second microscope image 610.

The embodiments of this disclosure enable dynamic control and flexible content sharing across multiple displays attached to the surgical microscope. For example, contents of the smaller control touchscreen and an alternative larger monitor (mounted on a flexible arm) can be swapped and controlled on either screen, allowing the surgical team to choose the optimal display for viewing the surgical field, adjusting microscope settings, adjusting camera and scene file settings, reviewing, selecting, or exporting media content, and conducting training sessions with enhanced visibility for all participants.

By allowing content and control interfaces to move across displays without interrupting workflow, this solution enhances accessibility, visibility, and usability in the surgical environment. The key principle is the seamless transfer and control of content across multiple displays without disrupting the workflow.

Embodiments of this disclosure integrate a software and hardware interface that allows the content and control functionalities of the multiple touchscreens and to be swapped and shared. This system ensures that all functionalities are accessible from either displays, enhancing user experience, ergonomic comfort, training efficiency, and collaborative work. Therefore, embodiments comprise a swappable content control interface with software to facilitates real-time swapping of display content between the monitors. Additionally or alternatively, embodiments comprise simple, intuitive GUIs to enable users to switch content with minimal effort. The microscope's interface can be accessed on either the small touchscreen or the larger monitor(s) by dynamically transferring content and control functionality between the two displays. This feature allows operators to control microscope settings, adjust system parameters, and view or export media content on whichever display is most accessible and suitable for the task.

Embodiments of this disclosure comprise flexible display options for multi-user scenarios. Therefore, a larger monitor, mounted on a flexible arm, which can be positioned for optimal group viewing during training sessions, allowing the control interface or media review functionalities to be displayed for better visibility. A control touchscreen can also show the live surgical view, providing an adaptable solution for different user needs, e.g., when space near the primary surgical area is constrained.

Embodiments of this disclosure comprise seamless workflow integration. Therefore, both display monitors may have touch capabilities, allowing for seamless control irrespective of which display is active. The content transfer between monitors is implemented to be fast and non-disruptive, ensuring a seamless switch between displays without workflow interruptions. The control options are available on either displays, allowing users to adjust settings or manage media on the fly, regardless of the display in use.

Embodiments of this disclosure comprise real-time content swapping. Thereby users can instantly switch content between the two displays, ensuring that the required information is always presented on the most convenient screen. Additionally or alternatively, embodiments may comprise enhanced viewing and control. Both displays can be used interchangeably for any function, be it surgical field viewing, system settings adjustments, or media file management. Embodiments may be configured for improved collaboration. A larger monitor can be used for group viewing and training, while still controlling the microscope settings and other functionalities.

Embodiments of this disclosure comprise wireless portable interface controls. A wireless version of the control interface could be operated from a tablet device, allowing even more flexible control by enabling settings adjustments from anywhere in the room. Additionally or alternatively, embodiments comprise voice-activated display switching. Voice commands may be integrated to facilitate hands-free content swapping, further enhancing usability in scenarios where the operator's hands are occupied. Additionally or alternatively embodiments may be configured for footswitch/handles-activated display switching. Footswitch/handle commands could be integrated to facilitate content swapping, further enhancing usability in scenarios where the operator's is executing a surgery.

Embodiments of this disclosure comprise workflow integration. Via a pre-operation setup displays may be configured based on user preference and operational requirements. Additionally embodiments may be configurable during operation. Users can dynamically switch content between displays to suit real-time needs, such as switching the live surgical view to the larger monitor for team observation while adjusting settings on the smaller touchscreen. Additionally or alternatively, embodiments may be configured for post-operation. This may facilitate media review and export on the larger display, enabling easier collaboration and documentation.

Embodiments of this disclosure comprise may improve the ergonomics, usability, and efficiency of surgical microscope systems by enabling flexible display control, enhancing the user experience in surgical, training, and media review workflows.

Some embodiments relate to a microscope comprising a system as described in connection with one or more of the Figs. 1 to 6. Alternatively, a microscope may be part of or connected to a system as described in connection with one or more of the Figs. 1 to 6.

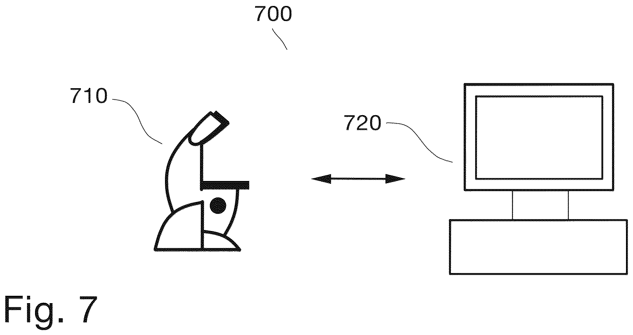

Fig. 7 shows a schematic illustration of a system 700 configured to perform a method described herein. The system 700 comprises a microscope 710 and a computer system 720. The microscope 710 is configured to take images and is connected to the computer system 720. The computer system 720 is configured to execute at least a part of a method described herein. The computer system 720 may be configured to execute a machine learning algorithm. The computer system 720 and microscope 710 may be separate entities but can also be integrated together in one common housing. The computer system 720 may be part of a central processing system of the microscope 710 and/or the computer system 720 may be part of a subcomponent of the microscope 710, such as a sensor, an actor, a camera or an illumination unit, etc. of the microscope 710.

The computer system 720 may be a local computer device (e.g. personal computer, laptop, tablet computer or mobile phone) with one or more processors and one or more storage devices or may be a distributed computer system (e.g. a cloud computing system with one or more processors and one or more storage devices distributed at various locations, for example, at a local client and/or one or more remote server farms and/or data centers). The computer system 720 may comprise any circuit or combination of circuits. In one embodiment, the computer system 720 may include one or more processors which can be of any type. As used herein, processor may mean any type of computational circuit, such as but not limited to a microprocessor, a microcontroller, a complex instruction set computing (CISC) microprocessor, a reduced instruction set computing (RISC) microprocessor, a very long instruction word (VLIW) microprocessor, a graphics processor, a digital signal processor (DSP), multiple core processor, a field programmable gate array (FPGA), for example, of a microscope or a microscope component (e.g. camera) or any other type of processor or processing circuit. Other types of circuits that may be included in the computer system 720 may be a custom circuit, an application-specific integrated circuit (ASIC), or the like, such as, for example, one or more circuits (such as a communication circuit) for use in wireless devices like mobile telephones, tablet computers, laptop computers, two-way radios, and similar electronic systems. The computer system 720 may include one or more storage devices, which may include one or more memory elements suitable to the particular application, such as a main memory in the form of random access memory (RAM), one or more hard drives, and/or one or more drives that handle removable media such as compact disks (CD), flash memory cards, digital video disk (DVD), and the like. The computer system 720 may also include a display device, one or more speakers, and a keyboard and/or controller, which can include a mouse, trackball, touch screen, voice-recognition device, or any other device that permits a system user to input information into and receive information from the computer system 720.

Some or all of the method steps may be executed by (or using) a hardware apparatus, like for example, a processor, a microprocessor, a programmable computer or an electronic circuit. In some embodiments, some one or more of the most important method steps may be executed by such an apparatus.

Depending on certain implementation requirements, embodiments of the invention can be implemented in hardware or in software. The implementation can be performed using a non-transitory storage medium such as a digital storage medium, for example a floppy disc, a DVD, a Blu-Ray, a CD, a ROM, a PROM, and EPROM, an EEPROM or a FLASH memory, having electronically readable control signals stored thereon, which cooperate (or are capable of cooperating) with a programmable computer system such that the respective method is performed. Therefore, the digital storage medium may be computer readable.

Some embodiments according to the invention comprise a data carrier having electronically readable control signals, which are capable of cooperating with a programmable computer system, such that one of the methods described herein is performed.

Generally, embodiments of the present invention can be implemented as a computer program product with a program code, the program code being operative for performing one of the methods when the computer program product runs on a computer. The program code may, for example, be stored on a machine readable carrier.

Other embodiments comprise the computer program for performing one of the methods described herein, stored on a machine readable carrier.

In other words, an embodiment of the present invention is, therefore, a computer program having a program code for performing one of the methods described herein, when the computer program runs on a computer.

A further embodiment of the present invention is, therefore, a storage medium (or a data carrier, or a computer-readable medium) comprising, stored thereon, the computer program for performing one of the methods described herein when it is performed by a processor. The data carrier, the digital storage medium or the recorded medium are typically tangible and/or non-transitionary. A further embodiment of the present invention is an apparatus as described herein comprising a processor and the storage medium.

A further embodiment of the invention is, therefore, a data stream or a sequence of signals representing the computer program for performing one of the methods described herein. The data stream or the sequence of signals may, for example, be configured to be transferred via a data communication connection, for example, via the internet.

A further embodiment comprises a processing means, for example, a computer or a programmable logic device, configured to, or adapted to, perform one of the methods described herein.

A further embodiment comprises a computer having installed thereon the computer program for performing one of the methods described herein.

A further embodiment according to the invention comprises an apparatus or a system configured to transfer (for example, electronically or optically) a computer program for performing one of the methods described herein to a receiver. The receiver may, for example, be a computer, a mobile device, a memory device or the like. The apparatus or system may, for example, comprise a file server for transferring the computer program to the receiver.

In some embodiments, a programmable logic device (for example, a field programmable gate array) may be used to perform some or all of the functionalities of the methods described herein. In some embodiments, a field programmable gate array may cooperate with a microprocessor in order to perform one of the methods described herein. Generally, the methods are preferably performed by any hardware apparatus.

### List of reference signs

- 100: operating room microscope system
- 102: operating table
- 106: surgeon
- 110: articulated microscope
- 112: microscope arm
- 114: microscope optical system
- 120: touch screen display
- 122: first camera image
- 124: selected information on first display
- 130: second display
- 132: second camera image
- 134: audience
- 200: first camera image
- 202: microscope image
- 210: GUI bottom bar
- 211: footswitch overlay configuration
- 212: image enhancement configuration
- 213: 2D 3D selection
- 214: OCT selection
- 215: swap screen
- 216: HDMI switch
- 220: GUI sidebar
- 230: subimage for first camera image
- 300: GUI for second display
- 302: main menu
- 304: scene menu
- 310: swap screen element
- 400: operating room
- 500: operating room
- 510: second image
- 512: additional information
- 520: first image and GUI
- 522: additional
- 600: operating room
- 610: microscope image
- 612: subimage
- 622: microscope image
- 700: system
- 710: microscope
- 720: computer system

## Claims

1. Device for displaying surgical images,
configured to:
- determine, via a content function, a first camera image (122) and a second camera image (132) based on a camera image of a surgical device (114);
- determine, via a control function, one or more graphical user interfaces, GUIs, (124) to control the surgical device and/or the content function;
- assign to a first display (120), in particular a touch screen display, the first camera image (122) and a GUI (124) concurrently;
- assign to a second display (130) the second camera image (132) and/or a GUI.

2. The device according to the preceding claim,
configured to:
- assign, by the content function, from one display to the other display the respective camera image (130, 132) and/or the respective GUI.

3. The device according to one of the preceding claims,
configured to:
- determine, by the content function, the first camera image (122) in a first resolution; and
- determine, by the content function, the second camera image (132) in a second resolution.

4. The device according to one of the preceding claims,
configured to:
- provide, by the content function, the first camera image to the first display within an image stream (122) with a first refresh rate;
- provide, by the content function, the second camera image to the second display within an image stream (132) with a second refresh rate, wherein the first refresh rate is different from the second refresh rate.

5. The device according to one of the preceding claims,
configured to one or more of the following:
- provide, by the content function, one or more camera images (122, 132) to a storage for later use;
- obtain, by the content function, one or more camera images from a storage for current use;
- provide, by the control function, one or more GUI-user-interactions to a storage for later use;
- obtain, by the control function, one or more GUI-user-interactions from a storage for current use.

6. The device according to one of the preceding claims,
configured to:
- assign to the second display (130), by the content function, the second camera image (132) and, by the control function, a GUI with a functionality of the GUI that is assigned to the first display (120).

7. The device according to one of the preceding claims,
configured to:
- assign, by the control function, to the second display (130) a GUI, which is different from the GUI assigned to the first display (120).

8. The device according to one of the preceding claims,
configured to:
- assign, by the control function, an image of the GUI and/or a GUI-user-interaction of the first display (120) to the second display (130).

9. The device according to one of the preceding claims,
configured to:
- to provide the first camera image (122) directly from the camera (114) to the first display (120) and/or to the second display (130).

10. The device according to one of the preceding claims,
configured to:
- receive a selection information (124), by the control function, of an object and/or an area indicated on one of the first display (120) or the second display (130);
- provide the selected object and/or area (124) for display, by the content function, to the respective other of the first display and/or the second display.

11. The device according to one of the preceding claims,
configured to:
- receive a selection information of an object and/or an area indicated by a user on one of the first display (120) or the second display;
- indicate the selected object and/or area (124) on the respective other of the first display and/or the second display (130).

12. The device according to one of the preceding claims,
configured to:
- display a structure on the second display indicated on the first display;
- display an area of interest in a second image;
- display a past part of a surgery for replay.

13. A system (100) for displaying surgical images,
comprising:
- a device according to one of the preceding claims,
- a first display, in particular a touch screen display (120);
- a second display (130);
- an input means;
configured to:
- assign results of a content function and/or of a control function of the device according to one of the preceding claims based on an information provided by the input means;
wherein the input means comprise one or more of:
- a haptic input means, in particular a footswitch;
- a remote control;
- a microphone;
- a camera.

14. A method for displaying surgical images,
comprising the steps:
- determining a first camera image (122) and a second camera image (132) based on a camera image of a surgical device (114);
- determining one or more graphical user interfaces, GUIs, (124) to control the surgical device and/or the determination of the first and/or second camera image;
- assigning to a first display (120), in particular a touch screen display, the first camera image (122) and a GUI (124) concurrently;
- assigning to a second display (130) the second camera image (132) and/or a GUI.

15. Computer program with a program code
for operating a device, a system and/or for executing a method according to one of the preceding claims, when the computer program is run on a processor.
